# EUROPEAN PATENT SPECIFICATION

(11) **EP 3 870 213 B1**
(45) Date of publication and mention of the grant of the patent: **07.06.2023**
(21) Application number: 19801228.8
(22) Date of filing: 25.10.2019
(51) Int. Cl.: A61K 38/26, A61K 9/00, A61K 47/18, A61K 47/10, A61P 3/10, A61P 3/04

(54) **STABLE SEMAGLUTIDE COMPOSITIONS AND USES THEREOF**
STABILE SEMAGLUTID-ZUSAMMENSETZUNGEN UND VERWENDUNGEN DAVON
COMPOSITIONS STABLES DU SÉMAGLUTIDE ET LEURS UTILISATIONS

(30) Priority: 26.10.2018 EP 18202801; 20.02.2019 EP 19158226
(43) Date of publication of application: 01.09.2021
(73) Proprietor: Novo Nordisk A/S, 2880 Bagsværd (DK)
(72) Inventor: ENGELUND, Dorthe, Kot, 2880 Bagsværd (DK); JENSEN, Søren, Skov, 2880 Bagsværd (DK); LUNDQVIST, Joakim, 2880 Bagsværd (DK)
(86) International application number: PCT/EP2019/079214
(87) International publication number: WO 2020/084126

(56) References cited:
- WO-A1-2016/038521
- WO-A1-2017/186896
- WO-A1-2018/115901
- WO-A2-2005/049061

## Description

The present invention relates to the field of pharmaceutical compositions comprising the GLP-1 peptide semaglutide and uses thereof.

### BACKGROUND

GLP-1 peptides are known to be prone to develop lack of stability in liquid solutions, for example lack of physical or chemical stability.

Thus, liquid pharmaceutical formulations comprising GLP-1 peptides with even better stability are desired. Such improved stability may be physical and/or chemical stability and may lead to an improved shelf-life of the pharmaceutical formulation.

### SUMMARY

The invention relates to liquid pharmaceutical compositions comprising semaglutide and histidine. In some embodiments, the invention relates to kits comprising the pharmaceutical composition as defined herein. In some embodiments, the invention relates to the pharmaceutical composition as defined herein for use in medicine. In some embodiments, the invention relates to a liquid pharmaceutical composition comprising semaglutide, an isotonic agent and histidine, wherein the concentration of the histidine is 0.5-100 mM and wherein the pH of the composition is in the range of 6.0-10.0. In some embodiments, the invention relates to a kit comprising a liquid pharmaceutical composition comprising semaglutide, an isotonic agent and histidine, wherein the concentration of the histidine is 0.5-100 mM and wherein the pH of the composition is in the range of 6.0-10.0, and instructions for use.

### DESCRIPTION

Liquid compositions comprising glucagon-like peptide 1 (GLP-1) peptides are known to be prone to develop lack of stability e.g. during storage and/or when exposed to light. During storage of a liquid pharmaceutical composition comprising semaglutide e.g. in a pre-filled syringe or in a cartridge, an increase in formation of impurities is observed over time.

The present inventors have observed that when a stabilizer, such as histidine is present in the liquid composition, low content of high molecular weight proteins (HMWPs) and other impurities are formed. Thus, the composition comprising stabilizer according to the present invention has improved chemical and/or physical stability. The improved stability results in benefits for the patient in form of a longer shelf-life and a longer in-use period.

The stabilizer is histidine. The composition comprises histidine in a concentration of 0.5-100 mM, such as 1-50 mM or 5-15 mM. In some embodiments, the composition comprises 0.01-10 mg/ml semaglutide. In some embodiments, the composition optionally comprises phenol, e.g. the composition comprises up to 10 mg/ml phenol, such as 5.5 mg/ml. In some embodiments, the composition comprises less than 0.01 % (w/w) phenol, such as no phenol. In some embodiments, the composition comprises no more than 0.01 % (w/w) phenol, such as no phenol. In some embodiments, the composition has a pH in the range of 6.0-10.0, such as 7.0-7.8.

Unless otherwise indicated in the specification, terms presented in singular form also include the plural situation.

### Pharmaceutical compositions

The terms "pharmaceutical composition" and "composition" are used interchangeably herein and refer to pharmaceutical compositions suitable for administration to a subject in need thereof.

In some embodiments, the composition of the invention comprises 0.01-100 mg/ml semaglutide. In some embodiments, the composition of the invention comprises 0.1-50 mg/ml, such as 0.5-25 mg/ml or 1-15 mg/ml, semaglutide. In some embodiments, the composition of the invention comprises 0.1-10 mg/ml, such as 0.5-5 mg/ml or 1-2 mg/ml, semaglutide. In some embodiments, the composition of the invention comprises 0.5 mg/ml semaglutide.

The compositions of the invention comprise a stabilizer. The term stabilizer refers herein to a compound minimizing the formation of impurities in the composition, such as HMWPs or other non-desirable impurities generated during storage as compared to a composition without the presence of the stabilizer. The stabilizer is histidine. HMWPs refers to high molecular weight proteins and may be determined as described in Assay(I) under General Methods and Characterisation. The term 'other non-desirable impurities generated during storage' refers to 'hydrophobic impurities 1' or 'hydrophobic impurities 2' and may be determined as described in Assay(ll) under General Methods and Characterisation.

In some embodiments, the composition of the invention comprises histidine as stabilizer in a concentration of 1-50 mM, such as 5-20 mM. In some embodiments, the composition comprises 0.5-15 mM histidine as stabilizer. In some embodiments, the composition of the invention comprises 10 mM histidine. In a specific embodiment, the stabilizer is histidine in a concentration of 10 mM.

The composition of the invention has a pH in the range of pH 6-10. In some embodiments, the composition of the invention has a pH in the range of pH 6.5-8-5, such as pH 7.0-7.8. In a particular embodiment, the pH of the composition is 7.4.

In some embodiments, the composition of the invention comprises one or more pharmaceutically acceptable excipients.

The composition of the invention comprises an isotonic agent. In some embodiments, the isotonic agent is 1,2-propanediol (propylene glycol).

In some embodiments, the composition of the invention comprises a buffer, such as phosphate buffer, TRIS, citrate, or no buffer. In some embodiments, the phosphate buffer is a sodium phosphate buffer, such as disodium hydrogen phosphate dihydrate. In some embodiments, histidine added as stabilizer, may also act as a buffer.

In some embodiments, the composition of the invention optionally comprises a preservative e.g. phenol in a concentration of up to 10 mg/ml, such as 5.5 mg/ml phenol. In some embodiments, the composition comprises phenol in a concentration of 0.1 mg/ml to 5.5 mg/ml. In some embodiments, the composition of the invention comprises no preservative, such as no phenol. In some embodiments, the liquid composition comprises less than 0.01% (w/w) phenol, such as no phenol. In some embodiments, the liquid composition comprises no more than 0.01 % (w/w) phenol, such as no phenol.

In some embodiments, the composition of the invention is a liquid composition in the form of a solution, such as an aqueous solution, i.e. comprising water. In some embodiments, the term "aqueous solution" as used herein refers to a solution comprising at least 60 % (w/w) water. In some embodiments, the aqueous solution comprises 60-99 % (w/w) water. In some embodiments, the aqueous solution comprises at least 75 % (w/w) water, such as at least 80 % (w/w) water or at least 85 % (w/w) water. In some embodiments, the aqueous solution comprises at least 90 % (w/w) water, such as at least 92 % (w/w) water or at least 94 % (w/w) water.

The composition comprises semaglutide, an isotonic agent, and histidine. In some embodiments, the composition comprises semaglutide, an isotonic agent, and histidine, wherein the concentration of histidine is 0.5-100 mM and wherein the pH of the composition is in the range of 6.0-10.0. In some embodiment, the composition comprises semaglutide in a concentration of 0.5-15 mg/ml, histidine in a concentration of 0.5-100 mM, and an isotonic agent, wherein the pH of the composition is in the range of 6.0-10.0. In some embodiment, the composition comprises semaglutide in a concentration of 0.5-15 mg/ml, histidine in a concentration of 0.5-100 mM, and propylene glycol, wherein the pH of the composition is in the range of 6.0-10.0. In some embodiment, the composition comprises semaglutide in a concentration of 0.5-15 mg/ml, histidine in a concentration of 0.5-15 mM, and propylene glycol, wherein the pH of the composition is in the range of 6.0-10.0. In some embodiment, the composition comprises semaglutide in a concentration of 0.5-15 mg/ml, histidine in a concentration of 0.5-15 mM, a phosphate buffer and propylene glycol, wherein the pH of the composition is in the range of 6.0-10.0.

### Semaglutide

The GLP-1 peptide semaglutide may be prepared as described in WO2006/097537, Example 4. Semaglutide is also known as N^{6,26}-{18-[N-(17-carboxyheptadecanoyl)-L-γ-glutamyl]-10-oxo-3,6,12,15-tetraoxa-9,18-diazaoctadecanoyl}-[8-(2-amino-2-propanoic acid), 34-L-arginine]human glucagon-like peptide 1(7-37), see WHO Drug Information Vol. 24, No. 1, 2010.

In some embodiments, semaglutide may be present in the composition in its fully or partly ionised form; for example one or more carboxylic acid groups (-COOH) may be deprotonated into the carboxylate group (-COO⁻) and/or one or more amino groups (-NH₂) may be protonated into the -NH₃⁺ groups. In some embodiments, semaglutide is added to the composition in the form of a salt.

### Administration and kits

The composition of the invention is for parenteral administration. In some embodiments, the composition is for subcutaneous administration, e.g. for administration by means of a syringe, optionally a pre-filled syringe, such as a pre-filled syringe with a staked needle. In some embodiments, the means for administration is a durable pen or a pre-filled pen.

In some embodiments, the composition of the invention is for administration once weekly. In some embodiments, the composition of the invention is for administration once daily, once every second or once every third day.

In some embodiments, the invention relates to a kit comprising the pharmaceutical composition as defined herein and instructions for use. In some embodiments, the instructions for use comprise the package insert of a drug.

In some embodiments, the invention relates to a kit comprising the pharmaceutical composition as defined herein and an injection device. In some embodiments, the pharmaceutical composition is in a pre-filled syringe or a cartridge that are inserted into the injection device. An example of a pre-filled syringe is a pre-filled syringe with a staked needle, such as a glass syringe or a polymer syringe. In some embodiments, the pre-filled syringe with a staked needle is a glass syringe. In some embodiments, the pre-filled syringe with a staked needle is a polymeric syringe. In some embodiments, the injection device is a durable pen or a pre-filled pen. Examples of durable pens are NovoPen^{®}4 or NovoPen^{®}5 (both from Novo Nordisk A/S, Denmark). An example of a prefilled pen is FlexPen^{®} (Novo Nordisk A/S, Denmark).

### Indications

In some embodiments, the compositions of the invention are for use in medicine. In some embodiments, the composition of the invention may be used for the following medical treatments:
(i) prevention and/or treatment of all forms of diabetes, such as hyperglycaemia, type 2 diabetes, impaired glucose tolerance, type 1 diabetes, non-insulin dependent diabetes, MODY (maturity onset diabetes of the young), gestational diabetes, and/or for reduction of HbA1c;
(ii) delaying or preventing diabetic disease progression, such as progression in type 2 diabetes, delaying the progression of impaired glucose tolerance (IGT) to insulin requiring type 2 diabetes, and/or delaying the progression of non-insulin requiring type 2 diabetes to insulin requiring type 2 diabetes;
(iii) prevention and/or treatment of eating disorders, such as obesity, e.g. by decreasing food intake, reducing body weight, suppressing appetite, inducing satiety; treating or preventing binge eating disorder, bulimia nervosa, and/or obesity induced by administration of an antipsychotic or a steroid; reduction of gastric motility; and/or delaying gastric emptying.
(iv) prevention and/or treatment of liver disorders, such as hepatic steatosis, non-alcoholic fatty liver disease (NAFLD), non-alcoholic steatohepatitis (NASH), liver inflammation or fatty liver.

In some embodiments the indication is (i). In some embodiments, the indication is (ii). In a still further particular aspect the indication is (iii). In some embodiment, the indication is (iv). In some embodiments, the indication is type 2 diabetes and/or obesity.

In some embodiments, the method or use comprises prevention, treatment, reduction and/or induction in one or more diseases or conditions defined herein. In some embodiments, the indication is (i) and (iii). In some embodiments, the indication is (ii) and (iii). In some embodiments, the invention comprises administration of an effective amount of a GLP-1 peptide. In some embodiments, the invention relates to administration of an effective amount of a GLP-1 peptide. In a particular embodiment, the invention relates to administration of an effective amount of semaglutide.

Generally, all subjects suffering from obesity are also considered to be suffering from overweight. In some embodiments, the invention relates to a method for treatment or prevention of obesity. In some embodiments, the invention relates to use of the composition for treatment or prevention of obesity. In some embodiments, the subject suffering from obesity is human, such as an adult human or a paediatric human (including infants, children, and adolescents). Body mass index (BMI) is a measure of body fat based on height and weight. The formula for calculation is BMI = weight in kilograms/height in meters². A human subject suffering from obesity may have a BMI of ≥30; this subject may also be referred to as obese. In some embodiments, the human subject suffering from obesity may have a BMI of ≥35 or a BMI in the range of ≥30 to <40. In some embodiments, the obesity is severe obesity or morbid obesity, wherein the human subject may have a BMI of ≥40.

In some embodiments, the invention relates to a method for treatment or prevention of overweight, optionally in the presence of at least one weight-related comorbidity. In some embodiments, the invention relates to use of the composition for treatment or prevention of overweight, optionally in the presence of at least one weight-related comorbidity. In some embodiments, the subject suffering from overweight is human, such as an adult human or a paediatric human (including infants, children, and adolescents). In some embodiments, a human subject suffering from overweight may have a BMI of ≥25, such as a BMI of ≥27. In some embodiments, a human subject suffering from overweight has a BMI in the range of 25 to <30 or in the range of 27 to <30. In some embodiments, the weight-related comorbidity is selected from the group consisting of hypertension, diabetes (such as type 2 diabetes), dyslipidaemia, high cholesterol, and obstructive sleep apnoea.

In some embodiments, the invention relates to a method for reduction of body weight. In some embodiments, the invention relates to use of the composition for reduction of body weight. A human to be subjected to reduction of body weight according to the present invention may have a BMI of ≥25, such as a BMI of ≥27 or a BMI of ≥30. In some embodiments, the human to be subjected to reduction of body weight according to the present invention may have a BMI of ≥35 or a BMI of ≥40. The term "reduction of body weight" may include treatment or prevention of obesity and/or overweight.

In some embodiments, as used herein, specific values given in relation to numbers or intervals may be understood as the specific value or as about the specific value (e.g. plus or minus 10 percent of the specific value).

### Examples

### General Methods and Characterisation

### Preparation of semaglutide compositions:

Unless otherwise noted, compositions of semaglutide were prepared by dissolving buffer (disodium hydrogen phosphate dihydrate), isotonic agent (propylene glycol), phenol (optionally) and optionally a stabilizer (histidine) in water. Semaglutide was dissolved therein, pH was adjusted to 7.4 using sodium hydroxide and/or hydrochloric acid, and the composition was finally sterilised by filtration through a 0.22 µm sterile filter. The compositions comprising semaglutide and histidine were added to a pre-filled syringe (Ompi #7600002.8506) or a cartridge.

### Assay (I): Determination of High Molecular Weight Proteins (HMWP) content of semaglutide compositions

Determination of HMWP content was performed using size exclusion chromatography (SE-HPLC) using a Waters Insulin HMWP column with a mobile phase of sodium chloride, sodium phosphate, phosphoric acid and isopropanol, isocratic elution and detection at 280 nm. Content of HMWP is given in % as the combined area of chromatographic peaks eluting earlier than the semaglutide monomer peak (i.e. HMWP peaks), relative to the total area of HMWP and semaglutide monomer peaks. The results are presented as absolute values and/or as % increase per month.

### Assay (II): Determination of Hydrophobic Impurities 1 and 2 of semaglutide compositions

Determination of Hydrophobic Impurities 1 and 2 of the semaglutide compositions was performed using reversed phase high performance liquid chromatography (RP-HPLC). The RP-HPLC method was performed on a Kinetex C18 column starting with an isocratic elution followed by a gradient elution using eluent A (90% 0.09M phosphate buffer, pH 3.6 and 10% acetonitrile) and eluent B (60% acetonitrile and 20% isopropanol) from approx 50:50 to 10:90 and back to approx. 50:50 of A:B. Detection was performed at 210 nm.

Hydrophobic impurities 1 were calculated as the area of all peaks eluting between semaglutide and the start of the final gradient in the chromatogram relative to the total area of all peaks. Hydrophobic Impurities 2 were calculated as the area of all peaks eluting in the final gradient in the chromatogram relative to the total area of all peaks.

### Assay (III): Physical stability of semaglutide compositions assessed via ThT

The purpose of this assay is to assess the physical stability of a GLP-1 peptide in aqueous solution.

Low physical stability of a peptide or protein may lead to amyloid fibril formation. Fibrils are structurally well-ordered, filamentous macromolecular structures formed by aggregation of soluble proteins and dominated by beta-sheet structure. Mature fibrils are insoluble and are resistant to degradation. For the sake of drug product quality and patient safety, it is desirable to minimize and control fibrillation events in pharmaceutical compositions of therapeutic peptides and proteins. Protein aggregation, including fibrillation, can be assessed by visual inspection of a sample. Fibrillation can be assessed by the use of Thioflavine T (ThT), a small molecule indicator probe with a high specificity for fibrils. ThT has a distinct fluorescence signature when binding to fibrils compared to ThT in solution [Naiki et al. (1989) Anal. Biochem. 177, 244-249; LeVine (1999) Methods. Enzymol. 309, 274-284].

Formation of a partially folded intermediate of the peptide is suggested as a general initiating mechanism for fibrillation. A small amount of these intermediates nucleates to form a template onto which further intermediates may assembly and the fibrillation proceeds. The lag-time corresponds to the interval in which a critical amount of nuclei is generated and the apparent rate constant is the rate with which the fibril itself is formed. The lag-time described in a ThT assay performed on a plate reader is therefore considered indicative of the fibrillation tendency of a peptide composition in solution.

Before performing the assay, ThT was added to the samples from a stock solution in H₂O to a final concentration of 20 µM in samples. Sample aliquots of 200 µl of the composition comprising the GLP-1 peptide were placed in a 96 well microtiter plate (optical 0.4 mL black Thermo Scientific Nunc) with a glass bead (2.8-3.2 mm, Whitehouse Scientific) placed in each well. Usually, eight replicas of each sample were placed on the plate. The plate was sealed with sealing tape (Thermo Scientific Nunc).

Incubation at given temperature, shaking and measurement of the ThT fluorescence emission were performed in a BMG FLUOStar Omega or a BMG FLUOStar Optima. The plate was incubated at 40°C with double orbital shaking at 300 rpm with an amplitude of 2 mm. Fluorescence measurement was performed using excitation through a 450 nm filter and measurement of emission through a 480 nm filter. The plate was measured every 20 minutes for a desired period of time. Between each measurement, the plate was shaken and heated as described.

The threshold value was determined as the highest ThT fluorescence (in relative fluorescence units (RFU)) measured on the plate at time 1 h 13 min, plus 100 RFU. The threshold value was then used to calculate the lag time using the "time to threshold" method in the BMG FLUOstar software.

### Example 1:

Compositions comprising semaglutide were tested in this example. The tested compositions contained semaglutide (0.5 mg/ml), propylene glycol (18.5 mg/ml), disodium hydrogen phosphate dihydrate (1.42 mg/ml), and optionally histidine (10 mM) as specified in Table 1, at pH 7.4 in an aqueous solution. The compositions were prepared as described under General Methods and Characterisation and the semaglutide compositions, were added to a pre-filled syringe with staked needle (filling volume 0.5 ml). The filled syringes were stored at 30°C or 37°C and the chemical stability followed over time. Some compositions were exposed to artificial light for 96 hours at 1000 lux. Following exposure to light the chemical stability was followed during storage at 37°C (also referred to herein as "37°C+light"). Chemical stability was determined by measuring formation of HMWP as described in Assay(I) after storage at 30°C, 37°C and 37°C+light; the results are provided in Table 2. Formation of Hydrophobic Impurities 1 and 2 was determined as described by Assay(ll) after storage at 30°C, 37°C and 37°C+light; the results are provided in Tables 3 and 4.

The results given in Tables 2, 3 and 4 surprisingly show that chemical stability of semaglutide is improved when histidine is present in the composition both with and without exposure to light.

**Table 1. Compositions tested in Example 1**

| **Composition no.** | **Description** |
|---|---|
| 1 | no histidine |
| 2 | 10 mM histidine |

**Table 2. HMWP formation in semaglutide compositions under given conditions.**

| **Conditions** | **Composition no.** | **HMWP(%)** | | | |
|---|---|---|---|---|---|
| | | **0 weeks** | **4 weeks** | **8 weeks** | **12 weeks** |
| 30°C | 1 (no histidine) | 0.1 | 0.3 | 0.4 | - |
| | 2 (10 mM histidine) | 0.1 | 0.2 | 0.2 | - |
| 37°C | 1 (no histidine) | 0.1 | 0.4 | 0.7 | 0.9 |
| | 2 (10 mM histidine) | 0.1 | 0.2 | 0.3 | 0.4 |
| 37°C + light | 1 (no histidine) | 0.2 | 0.8 | 0.8 | - |
| | 2 (10 mM histidine) | 0.1 | 0.3 | 0.6 | - |

The results presented in Table 2 show that when histidine is present in the composition, less HMWPs are formed during storage. A lower HMWP concentration corresponds to a better stability, i.e. the composition is chemically more stable when histidine is present both with and without exposure to light.

**Table 3. Formation of Hydrophobic Impurities 2 in semaglutide compositions under given conditions.**

| **Conditions** | **Composition no.** | **Hydrophobic Impurities 2 (%)** | | | |
|---|---|---|---|---|---|
| | | **0 weeks** | **4 weeks** | **8 weeks** | **12 weeks** |
| 30°C | 1 (no histidine) | <0.1 | 0.2 | 0.7 | - |
| | 2 (10 mM histidine) | <0.1 | 0.1 | 0.2 | - |
| 37°C | 1 (no histidine) | <0.1 | 0.3 | 0.8 | 1.1 |
| | 2 (10 mM histidine) | <0.1 | 0.1 | 0.4 | 0.4 |
| 37°C+light | 1 (no histidine) | 0.1 | 0.8 | 0.8 | - |
| | 2 (10 mM histidine) | 0.1 | 0.4 | 0.5 | - |

The results presented in Table 3 show that when histidine is present in the composition, less Hydrophobic Impurities 2 are formed during storage at both 30°C, 37°C and 37°C+light, i.e. the composition is more stable when histidine is present.

**Table 4. Formation of Hydrophobic Impurities 1 in semaglutide compositions under given conditions.**

| **Conditions** | **Composition no.** | **Hydrophobic Impurities 1 (%)** | | | |
|---|---|---|---|---|---|
| | | **0 weeks** | **4 weeks** | **8 weeks** | **12 weeks** |
| 30°C | 1 (no histidine) | 1.6 | 2.3 | 2.8 | - |
| | 2 (10 mM histidine) | 1.5 | 1.8 | 2.1 | - |
| 37°C | 1 (no histidine) | 1.6 | 3.0 | 4.6 | 5.7 |
| | 2 (10 mM histidine) | 1.5 | 2.5 | 3.3 | 4.5 |
| 37°C+light | 1 (no histidine) | 2.3 | 5.0 | 6.1 | - |
| | 2 (10 mM histidine) | 1.6 | 2.7 | 3.6 | - |

The results presented in Table 4 show that when histidine is present in the composition, less Hydrophobic Impurities 1 are formed during storage at both 30°C, 37°C and 37°C+light, i.e. the composition is chemically more stable when histidine is present.

### Example 2:

Compositions comprising semaglutide were tested in this example. The tested compositions contained semaglutide (0.5 mg/ml), propylene glycol (18.5 mg/ml), disodium hydrogen phosphate dihydrate (1.42 mg/ml), phenol (0, 0.1 or 5.5 mg/ml) and optionally histidine (10 mM) as specified in Tables 5-9, at pH 7.4 in an aqueous solution. The compositions were prepared as described under General Methods and Characterisation and the semaglutide compositions, were added to a pre-filled syringe (PFS) with staked needle (filling volume 0.5 ml) or 1.5 ml cartridges (filling volume 1.5 ml). The filled syringes or cartridges were stored at 37°C and the chemical stability followed over time. Some compositions were exposed to artificial light for 96 hours at 1000 lux. Following exposure to light the chemical stability was followed during storage at 37°C. Chemical stability was determined by measuring formation of HMWP as described in Assay(I) after storage at 37°C and 37°C+light; the results are provided in Table 5. Formation of Hydrophobic Impurities 1 and 2 was determined as described by Assay(ll) after storage at 37°C and 37°C+light; the results are provided in Tables 6 and 7. An overview of results for chemical stability calculated as % increase per month is given in Table 8. Physical stability as expressed by Thioflavine T (ThT) assay was determined by Assay (III) described herein and results given in Table 9.

In line with the results of Example 1, these results show that chemical stability of semaglutide is improved when histidine is present in the composition both with and without exposure to light. An improved chemical stability in presence of histidine is also observed independent of the primary packaging (pre-filled syringe or cartridge).

Regarding physical stability the results presented in Table 9 show that a longer lag time is obtained for compositions with histidine in the presence of 5.5 mg/ml phenol with or without exposure to light, i.e. the composition is more physically stable when histidine is present.

**Table 5. Formation of HMWP in semaglutide compositions under given conditions**

| 10 mM Histidine (Yes/no) | Primary packaging (PFS/cartridge) | Phenol (mg/ml) | Exposure to light (Yes/no) | HMWP (%) during storage at 37°C (months) | | | |
|---|---|---|---|---|---|---|---|
| | | | | 0 | 1 | 2 | 3 |
| No | PFS | 0 | No | 0.1 | 0.6 | 0.8 | 0.9 |
| No | Cartridge | 0 | No | 0.1 | 0.2 | 0.3 | 0.5 |
| Yes | PFS | 0 | No | 0.1 | 0.2 | 0.7 | 0.5 |
| Yes | Cartridge | 0 | No | 0.1 | 0.2 | 0.3 | 0.5 |
| Yes | Cartridge | 0 | Yes | 0.2 | 0.3 | 0.5 | 0.7 |
| No | Cartridge | 5.5 | No | 0.2 | 2.0 | 3.9 | 5.8 |
| No | PFS | 5.5 | No | 0.2 | 2.3 | 4.4 | 6.2 |
| No | Cartridge | 5.5 | Yes | 0.4 | 2.3 | 4.1 | 6.2 |
| Yes | Cartridge | 5.5 | No | 0.1 | 0.3 | 0.6 | 1.3 |
| Yes | PFS | 5.5 | No | 0.1 | 0.4 | 1.0 | 2.0 |
| Yes | Cartridge | 5.5 | Yes | 0.2 | 0.4 | 1.0 | 1.8 |
| No | Cartridge | 0.1 | No | 0.1 | 0.2 | 0.4 | 0.5 |
| Yes | Cartridge | 0.1 | No | 0.1 | 0.2 | 0.3 | 0.5 |

**Table 6. Formation of Hydrophobic Impurities 2 in semaglutide compositions under given conditions**

| 10 mM Histidine (Yes/no) | Primary packaging (PFS/cartridge) | Phenol (mg/ml) | Exposure to light (Yes/no) | Hydrophobic impurities 2 (%) during storage at 37°C (months) | | | |
|---|---|---|---|---|---|---|---|
| | | | | 0 | 1 | 2 | 3 |
| No | PFS | 0 | No | 0.1 | 0.6 | 0.7 | 1.0 |
| No | Cartridge | 0 | No | <0.1 | 0.3 | 0.5 | 0.6 |
| Yes | PFS | 0 | No | <0.1 | 0.3 | 0.6 | 0.8 |
| Yes | Cartridge | 0 | No | <0.1 | 0.2 | 0.4 | 0.5 |
| Yes | Cartridge | 0 | Yes | 0.1 | 0.3 | 0.5 | 0.6 |
| No | Cartridge | 5.5 | No | <0.1 | 1.4 | 2.7 | 4.0 |
| No | PFS | 5.5 | No | 0.1 | 1.7 | 2.9 | 4.4 |
| No | Cartridge | 5.5 | Yes | 0.2 | 1.6 | 2.8 | 4.7 |
| Yes | Cartridge | 5.5 | No | <0.1 | 0.2 | 0.6 | 1.3 |
| Yes | PFS | 5.5 | No | <0.1 | 0.3 | 1.4 | 1.4 |
| Yes | Cartridge | 5.5 | Yes | 0.1 | 0.4 | 0.9 | 1.5 |
| No | Cartridge | 0.1 | No | <0.1 | 0.2 | 0.4 | 0.6 |
| Yes | Cartridge | 0.1 | No | <0.1 | 0.2 | 0.4 | 0.4 |

**Table 7. Formation of Hydrophobic Impurities 1 in semaglutide compositions under given conditions**

| 10 mM Histidine (Yes/no) | Primary packaging (PFS/cartridge) | Phenol (mg/ml) | Exposure to light (Yes/no) | Hydrophobic impurities 1 (%) during storage at 37°C (months) | | | |
|---|---|---|---|---|---|---|---|
| | | | | 0 | 1 | 2 | 3 |
| No | PFS | 0 | No | 1.6 | 3.0 | 4.5 | 5.8 |
| No | Cartridge | 0 | No | 1.6 | 2.8 | 4.3 | 5.6 |
| Yes | PFS | 0 | No | 1.5 | 2.5 | 3.7 | 5.0 |
| Yes | Cartridge | 0 | No | 1.5 | 2.4 | 3.7 | 4.8 |
| Yes | Cartridge | 0 | Yes | 1.5 | 2.5 | 3.8 | 4.9 |
| No | Cartridge | 5.5 | No | 1.7 | 3.2 | 5.3 | 6.7 |
| No | PFS | 5.5 | No | 1.7 | 3.4 | 5.5 | 6.9 |
| No | Cartridge | 5.5 | Yes | 1.7 | 3.4 | 5.5 | 6.9 |
| Yes | Cartridge | 5.5 | No | 1.6 | 2.6 | 4.0 | 5.4 |
| Yes | PFS | 5.5 | No | 1.6 | 2.7 | 4.4 | 5.4 |
| Yes | Cartridge | 5.5 | Yes | 1.6 | 2.7 | 4.2 | 5.5 |
| No | Cartridge | 0.1 | No | 1.5 | 2.8 | 4.4 | 5.6 |
| Yes | Cartridge | 0.1 | No | 1.5 | 2.5 | 3.8 | 4.8 |

**Table 8. Overview of chemical stability given as % increase per month for HMWP, Hydrophobic impurities 1 and Hydrophobic impurities 2**

| Primary packaging (PFS/cartridge) | Phenol (mg/ml) | 10 mM Histidine (Yes/no) | Exposure to Light (yes/no) | HMWP (%/month) | Hydrophobic impurities 1 (%/month) | Hydrophobic Impurities 2 (%/month) |
|---|---|---|---|---|---|---|
| Cartridge | 0 | No | No | 0.13 | 1.32 | 0.17 |
| Cartridge | 0 | Yes | No | 0.13 | 1.10 | 0.14 |
| Cartridge | 0 | Yes | Yes | 0.17 | 1.12 | 0.17 |
| Cartridge | 0.1 | No | No | 0.14 | 1.36 | 0.17 |
| Cartridge | 0.1 | Yes | No | 0.13 | 1.09 | 0.11 |
| Cartridge | 5.5 | No | No | 1.83 | 1.67 | 1.27 |
| Cartridge | 5.5 | No | Yes | 1.88 | 1.73 | 1.44 |
| Cartridge | 5.5 | Yes | No | 0.38 | 1.25 | 0.39 |
| Cartridge | 5.5 | Yes | Yes | 0.53 | 1.29 | 0.46 |
| PFS | 0 | No | No | 0.25 | 1.38 | 0.27 |
| PFS | 0 | Yes | No | 0.17 | 1.14 | 0.23 |
| PFS | 5.5 | No | No | 1.96 | 1.73 | 1.38 |
| PFS | 5.5 | Yes | No | 0.62 | 1.28 | 0.49 |

**Table 9. Physical stability of semaglutide compositions as expressed by Thioflavine T (ThT) assay. A longer lag time corresponds to a better physical stability.**

| 10 mM Histidine (Yes/no) | Primary packaging (PFS/cartridge) | Phenol (mg/ml) | Exposure to light (Yes/no) | Lag time (hours) |
|---|---|---|---|---|
| No | PFS | 0 | No | >166 |
| No | Cartridge | 0 | No | >166 |
| Yes | PFS | 0 | No | >166 |
| Yes | Cartridge | 0 | No | >166 |
| Yes | Cartridge | 0 | Yes | >166 |
| No | Cartridge | 5.5 | No | 100 |
| No | PFS | 5.5 | No | 97 |
| No | Cartridge | 5.5 | Yes | 50 |
| Yes | Cartridge | 5.5 | No | >166 |
| Yes | PFS | 5.5 | No | 149 |
| Yes | Cartridge | 5.5 | Yes | >166 |
| No | Cartridge | 0.1 | No | >166 |
| Yes | Cartridge | 0.1 | No | >166 |

| | | | | |
|---|---|---|---|---|
| Results are an average of 6 samples tested | | | | |

### Example 3:

Compositions comprising semaglutide were tested in this example. The tested compositions contained semaglutide (0.1, 0.5, 2.0, 5.0 and 10 mg/ml), propylene glycol (18.5 mg/ml), disodium hydrogen phosphate dihydrate (1.42 mg/ml) and optionally histidine (5 and 10 mM) as specified in Tables 10, at pH 7.4 in an aqueous solution. The compositions were prepared as described under General Methods and Characterisation and the semaglutide compositions, were added to a pre-filled syringe (PFS) with staked needle (filling volume 0.5 ml). The filled syringes were stored at 30°C for 3 months and the chemical stability followed over time. Chemical stability was determined by measuring formation of HMWP as described in Assay(I) after storage at 30°C. Formation of Hydrophobic Impurities 1 and 2 was determined as described by Assay(ll) after storage at 30°C. Results calculated as % increase per month are provided in Table 10.

**Table 10. Overview of chemical stability given as % increase per month for HMWP Hydrophobic impurities 1 and 2 during storage at 30°C.**

| Histidine (mM) | Semaglutide (mg/ml) | HMWP (%/month) | Hydrophobic impurities 1 (%/month) | Hydrophobic impurities 2 (%/month) |
|---|---|---|---|---|
| 0 | 0.5 | 0.35 | 0.71 | 0.37 |
| 0 | 5 | 0.19 | 0.46 | 0.19 |
| 0 | 10 | 0.16 | 0.44 | 0.22 |
| 5 | 0.5 | 0.11 | 0.41 | 0.17 |
| 10 | 0.1 | 0.08 | 0.41 | 0.09 |
| 10 | 0.5 | 0.10 | 0.36 | 0.09 |
| 10 | 2 | 0.08 | 0.40 | 0.10 |
| 10 | 5 | 0.10 | 0.40 | 0.10 |
| 10 | 10 | 0.12 | 0.39 | 0.18 |

The results show that chemical stability of semaglutide is improved when histidine is present in the composition independent of amount of semaglutide and independent of amount of histidine.

Similarly, compositions comprising 0.5 mg/ml semaglutide, 18.5 mg/ml propylene glycol, 1.42 mg/ml disodium hydrogen phosphate dihydrate with and without 10 mM histidine in aqueous solution at pH 7.4 were stored at 30 °C for three months in pre-filled syringes from different vendors. The results showed that the chemical stability of semaglutide was improved independently on which syringe/vendor was used.

## Claims

1. A liquid pharmaceutical composition comprising semaglutide, an isotonic agent and histidine, wherein the concentration of the histidine is 0.5-100 mM and wherein the pH of the composition is in the range of 6.0-10.0.

2. The composition according to claim 1, wherein the concentration of histidine is 0.5-15 mM.

3. The composition according to any one of the preceding claims, wherein the composition is an aqueous solution comprising at least 60% (w/w) water, such as at least 70% (w/w) water or at least 80% (w/w) water.

4. The composition according to any one of the preceding claims, wherein the concentration of semaglutide is 0.1-15 mg/mL of the composition.

5. The composition according to any one of the preceding claims, wherein the concentration of semaglutide is 0.1-10 mg/mL of the composition.

6. The composition according to any one of the preceding claims, wherein the composition further comprises one or more pharmaceutically acceptable excipients such as a buffer.

7. The composition according to any one of the preceding claims, wherein the composition comprises a phosphate buffer.

8. The composition according to any one of the preceding claims, wherein the composition comprises disodium hydrogen phosphate dihydrate as a buffer.

9. The composition according to any one of the preceding claims, wherein the isotonic agent is propylene glycol.

10. The composition according to any one of the preceding claims, wherein the composition is in a pre-filled syringe or a cartridge.

11. A kit comprising the pharmaceutical composition according to any one of the preceding claims and instructions for use.

12. The kit according to claim 11, wherein the kit comprises a pre-filled syringe for administration of the pharmaceutical composition to a subject.

13. The kit according to claim 11, wherein the kit comprises a durable pen or a pre-filled pen for administration of the pharmaceutical composition to a subject.

14. A pharmaceutical composition according to any one of claims 1-10 for use as a medicament.

15. The pharmaceutical composition according to claim 14 for use in the treatment of diabetes or obesity.

## Patentansprüche

1. Flüssige pharmazeutische Zusammensetzung, umfassend Semaglutid, ein isotonisches Mittel und Histidin, wobei die Konzentration des Histidins 0,5-100 mM beträgt und wobei der pH-Wert der Zusammensetzung im Bereich von 6,0-10,0 liegt.

2. Zusammensetzung nach Anspruch 1, wobei die Konzentration von Histidin 0,5-15 mM beträgt.

3. Zusammensetzung nach einem der vorhergehenden Ansprüche, wobei die Zusammensetzung eine wässrige Lösung ist, die zumindest 60 Gew.-% Wasser, wie zumindest 70 Gew.-% Wasser oder zumindest 80 Gew.-% Wasser umfasst.

4. Zusammensetzung nach einem der vorhergehenden Ansprüche, wobei die Konzentration von Semaglutid 0,1-15 mg/ml der Zusammensetzung beträgt.

5. Zusammensetzung nach einem der vorhergehenden Ansprüche, wobei die Konzentration von Semaglutid 0,1-10 mg/ml der Zusammensetzung beträgt.

6. Zusammensetzung nach einem der vorhergehenden Ansprüche, wobei die Zusammensetzung ferner einen oder mehrere pharmazeutisch unbedenkliche Hilfsstoffe, wie einen Puffer, umfasst.

7. Zusammensetzung nach einem der vorhergehenden Ansprüche, wobei die Zusammensetzung einen Phosphatpuffer umfasst.

8. Zusammensetzung nach einem der vorhergehenden Ansprüche, wobei die Zusammensetzung Dinatriumhydrogenphosphatdihydrat als Puffer umfasst.

9. Zusammensetzung nach einem der vorhergehenden Ansprüche, wobei das isotonische Mittel Propylenglykol ist.

10. Zusammensetzung nach einem der vorhergehenden Ansprüche, wobei die Zusammensetzung in einer vorgefüllten Spritze oder einer Patrone vorliegt.

11. Kit, umfassend die pharmazeutische Zusammensetzung nach einem der vorhergehenden Ansprüche und Gebrauchsanweisungen.

12. Kit nach Anspruch 11, wobei das Kit eine vorgefüllte Spritze zur Verabreichung der pharmazeutischen Zusammensetzung an einen Patienten umfasst.

13. Kit nach Anspruch 11, wobei das Kit einen dauerhaften Pen oder einen vorgefüllten Pen zur Verabreichung der pharmazeutischen Zusammensetzung an einen Patienten umfasst.

14. Pharmazeutische Zusammensetzung nach einem der Ansprüche 1-10 zur Verwendung als Medikament.

15. Pharmazeutische Zusammensetzung nach Anspruch 14 zur Verwendung bei der Behandlung von Diabetes oder Adipositas.

## Revendications

1. Composition pharmaceutique liquide comprenant du sémaglutide, un agent isotonique et de l'histidine, dans laquelle la concentration de l'histidine est de 0,5 à 100 mM et dans laquelle le pH de la composition est dans la gamme de 6,0 à 10,0.

2. Composition selon la revendication 1, dans laquelle la concentration d'histidine est de 0,5 à 15 mM.

3. Composition selon l'une quelconque des revendications précédentes, dans laquelle la composition est une solution aqueuse comprenant au moins 60 % (p/p) d'eau, telle qu'au moins 70 % (p/p) d'eau ou au moins 80 % (p/p) d'eau.

4. Composition selon l'une quelconque des revendications précédentes, dans laquelle la concentration de sémaglutide est de 0,1 à 15 mg/ml de la composition.

5. Composition selon l'une quelconque des revendications précédentes, dans laquelle la concentration de sémaglutide est de 0,1 à 10 mg/ml de la composition.

6. Composition selon l'une quelconque des revendications précédentes, dans laquelle la composition comprend en outre un ou plusieurs excipients pharmaceutiquement acceptables tels qu'un tampon.

7. Composition selon l'une quelconque des revendications précédentes, dans laquelle la composition comprend un tampon phosphate.

8. Composition selon l'une quelconque des revendications précédentes, dans laquelle la composition comprend du hydrogénophosphate de disodium dihydraté comme tampon.

9. Composition selon l'une quelconque des revendications précédentes, dans laquelle l'agent isotonique est le propylène glycol.

10. Composition selon l'une quelconque des revendications précédentes, dans laquelle la composition est une seringue pré-remplie ou une cartouche.

11. Kit comprenant la composition pharmaceutique selon l'une quelconque des revendications précédentes et le mode d'emploi.

12. Kit selon la revendication 11, dans lequel le kit comprend une seringue pré-remplie pour l'administration de la composition pharmaceutique à un sujet.

13. Kit selon la revendication 11, dans lequel le kit comprend un stylo durable ou un stylo pré-rempli pour l'administration de la composition pharmaceutique à un sujet.

14. Composition pharmaceutique selon l'une quelconque des revendications 1 à 10, pour une utilisation en tant que médicament.

15. Composition pharmaceutique selon la revendication 14 pour une utilisation dans le traitement du diabète ou de l'obésité.
